# EUROPEAN PATENT SPECIFICATION

(11) **EP 1 488 763 B1**
(45) Date of publication and mention of the grant of the patent: **14.11.2007**
(21) Application number: 04251635.1
(22) Date of filing: 22.03.2004
(51) Int. Cl.: A61F 2/06

(54) **Stent with attached sleeve marker**
Stent mit einem angefügten Marker in der Form einer Hülse
Stent muni d' un marqueur en forme de manchon

(30) Priority: 16.06.2003 US 463114
(43) Date of publication of application: 22.12.2004
(60) Divisional application: 07014230.2
(73) Proprietor: Endotex Interventional Systems, Inc., Cupertino, CA 95014 (US)
(72) Inventor: Yang, Yi, San Francisco, California 94116 (US); Ong, Ich, San Jose, California 95111 (US); Hogben, Scott, San Mateo, California 94403 (US)
(74) Representative: McLeish, Nicholas Alistair Maxwell

(56) References cited:
- EP-A- 1 255 507
- EP-A- 1 356 789
- EP-A- 1 362 564
- US-A- 6 022 374
- US-A1- 2004 015 229
- US-B1- 6 293 966

## Description

### FIELD OF THE INVENTION

This invention relates to prostheses devices for implantation within body lumens, generally called stents, and more particularly to stents with attached radiopaque markers.

### BACKGROUND OF THE INVENTION

Stents are generally tubular-shaped prostheses which are deployed in a blood vessel or other anatomical lumen. Stents are useful in the treatment of atherosclerotic stenosis in blood vessels, and are used to widen and circumferentially support and hold open a blood vessel that has been narrowed and occluded by vascular disease. These endoprostheses or stents generally consist of an expandable tubular member which has as small profile in a contracted state so that it can be delivered to the site by minimally invasive procedures such as percutenous transluminal angioplasty. Stents are also used in graft prostheses which are implanted within blood vessels to prevent vessel wall rupture, particularly in the aorta or other arteries which may be subject to aneurysm formation and/or severe atherosclerotic disease which may involve multiple stenoses.

A variety of structures are used as stents or intraluminal vascular grafts, including stents made of wire mesh or slotted tubular elements, coiled springs, helical wire coil, and coiled sheet and helical mesh stents such as, e.g., those described in U.S. Patent Nos. 5,824,054 and 6,425,915, among others. Stents are typically loaded into a catheter in a contracted state and percutaneously introduced and advanced to a treatment site within a blood vessel, and may be balloon-expandable or self-expanding.

In order to precisely place a stent at the desired location, it is necessary to identify the exact position of the stent within a blood vessel. One means used to accomplish this is to incorporate a radiopaque marker into the stent so that, through the use of fluoroscopy, the position of the stent within the blood vessel can be identified, both at the time of placement and during subsequent checkups. For example, U.S. Pat. No. 6,409,752 describes a stent having a waveform pattern formed from a flat sheet of material in which a radiopaque marker of gold, platinum, tungsten, or iridium is positioned in a circular eyelet opening formed through the thickness of the sheet at the ends of the stent. The marker is positioned in the eyelet by melting the marker material in place or by crimping or other fastening methods. U.S. Pat. No. 6,402,777 describes radiopaque markers for use in stents formed by compressing, welding, or fusing a rivet of gold, tantalum or platinum through an opening in the ends or edges of the stent. Other methods for enabling the precise identification of a stent location include coating or plating the stent edges as markers as described in, e.g., European Patent Application No. 95302708 and U.S. Pat. No. 6,086,604; filling hollow stent wire with radiopaque material; producing the stent itself from radiopaque material such as tantalum; and using thicker metal at the ends of the stent as described in US2002/0072792A1.

Given the importance of precise placement of a stent at the desired location within a blood vessel, there is a need for additional approaches to providing stents witch markers to aid in achieving proper stent placement for a variety of stent design.

EP1, 255, 507 describes a stent matrix to improve radiopacity in a stent, and to reduce trauma while improving stent anchozing, imported beads are mounted to stent precursor matrices. Specially attractive is to provide a ring of such beads on the end rings of stent structures featuring a cylindrical mid length section flanked by outwardly flared ends. The beads can be of the same or different material from that of the stent matrix. The beads can assist in drawing the stent into a sleeve of a delivery system. Beads can be used to reveal features of the stent away from an end ring, such as a fenestration in the stent cylinder. Beads can be fixed in a position mechanically or be wielding.

EP1,362,564 describes an intravascular stent device. An intravascular stent, or aneurysm cover, which may be used to occlude, or partially occlude, an aneurysm in the human brain, comprises a thin-walled skeletal tubular member formed of interconnected sinusoidal members to thereby form a pattern of cells along the skeletal tubular member. The stent also includes anchor members, which are comprised of a longitudinal leg member having a radiopaque coil disposed about the leg member, which serve to retain the stew during delivery of the stent.

### SUMMARY OF THE INVENTION

According to this invention, a marker in the shape of an open-ended sleeve is formed from radiopaque material, such as platinum or tantalum, for attachment to a strut at the edges of an endoprostheses or stent. The sleeve marker is slid over and around the terminating end of the stent strut and the marker is permanently secured to the strut by changing the shape of the marker or the strut or by providing a snap-fit mechanism for retaining the marker on the strut. For example, where the strut is provided with an indentation or slot opening through the strut, the marker may be crimped and permanently deformed so that a portion of the marker extends below the surface of the stent strut. Alternately, the sides of a slotted strut may be expanded to widen the profile of the strut distal to the marker to prevent subsequent removal of the marker from the strut. The profile of the terminal end of the strut may also be expanded or increased in size after the sleeve marker is placed on the strut by other ways, including by forming a weld ball in the end of the strut having a sufficient diameter to prevent the marker from being removed from the strut. Preferably, the terminal end of the strut is provided with a portion having an increased width that is adapted to contract slightly and then snap back into place as the sleeve marker slides over it, thereby retaining the sleeve marker in place on the terminal strut.

Sleeve markers according to this invention can be used with any type of stent or stent graft so long as the stent has strut portions over which the sleeve marker can be slid and secured into place. The marker can be formed of a variety of materials which are more radiopaque than the material forming the stent and which can be identified by fluoroscopy or other such techniques.

In summary, according to a first aspect of the invention, there is provided an endovascular stent, having a radiopaque marker, in accordance with appended claim 1.

According to a second aspect of the invention, there is provided a method of attaching a radiopaque marker to a stent in accordance with appended claim 10.

### BRIEF DESCRIPTION OF THE DRAWINGS

FIG. 1 shows a preferred illustrative embodiment of a mesh sheet for a stent of the present invention having terminating struts at its ends.

FIG. 2 shows an enlarged view of a portion of a terminating stent strut which has a slot along its length.

FIG. 3 shows a sleeve marker of the present invention prior to its attachment to the stent strut.

FIG. 4 shows a sleeve marker placed surrounding a slotted stent strut and crimped on one side into the slot of the strut.

FIG. 5 shows the sleeve marker crimped on two opposite sides into the slot of the stent strut.

FIG. 6 shows an alternate embodiment in which the sides of the slot of the stent strut have been expanded outward distal to a sleeve marker placed around the strut.

FIG. 7 shows a terminating slotted stent strut having a stop member against which a sleeve marker can be placed.

FIG. 8 shows a sleeve marker placed surrounding the terminating slotted stent strut of FIG. 7 with the sides of the slot expanded outward distal to the sleeve marker.

FIGS. 9a-9c show an alternate embodiment of a sleeve marker attached to a stent.

FIG. 10 shows a still further alternate embodiment of a sleeve marker attached to a stent.

### DETAILED DESCRIPTION OF THE PREFERRED EMBODIMENTS

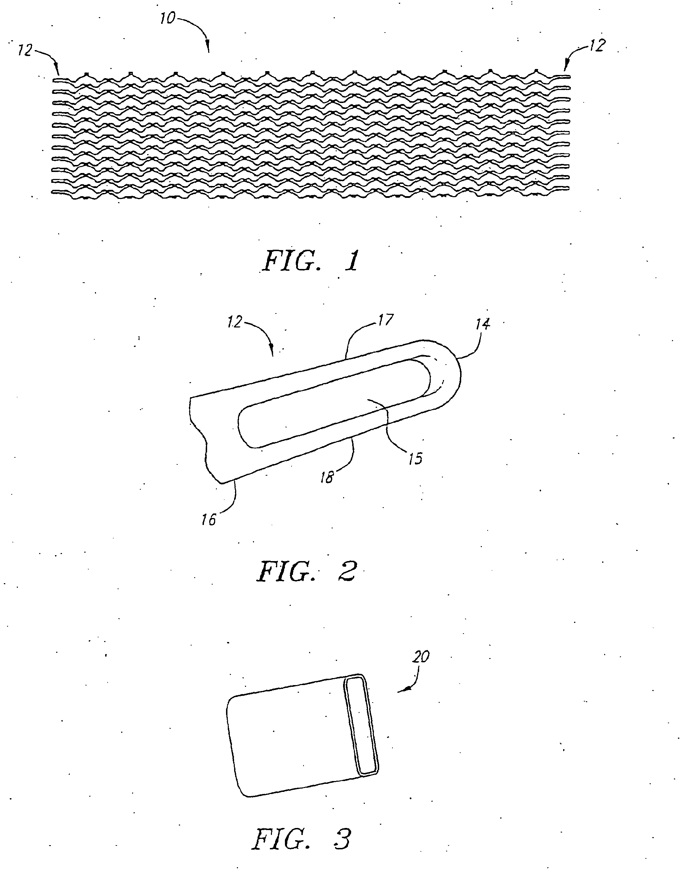

FIG. 1 shows a preferred illustrative embodiment of a stent **10** having terminating struts **12** at its edges to which sleeve markers can be attached in accordance with the present invention. As shown in FIG. 1, the terminating stent struts **12** are elongated portions which are formed as part of the stent, but are connected to the other portions of the stent only at one end and have a free terminal end. In a particularly preferred embodiment, the stent is a mesh coiled sheet stent formed of a mesh sheet as shown in FIG. 1 (shown prior to being coiled into the tube shape of the stent), having struts forming open expandable cells and having terminating struts at each end of the stent. Such a stent is similar to those described in United States Patent Application S/N 10/462876, entitled "Coiled-Sheet Stent With Flexible Mesh Design," assigned to Endotex Interventional Systems, Inc., and filed on the same date as the present application. The aforementioned patent application is hereby incorporated herein by reference in its entirety as if fully set forth herein. However, many other types of stents are also contemplated by this invention so long as the stent contains terminating struts to which sleeve markers can be attached. The stent and struts may be made of any suitable material, including a shape memory material such as Nitinol.

FIG. 2 shows an enlargement of an illustrative terminating stent strut **12.** FIG. 2 shows the free terminal end **14** of the strut and the opposite end **16** by which the strut is attached to the rest of the stent. Stent strut **12** shown in FIG. 2 is a slotted strut which has an elongated slot **15** formed in the strut between the strut's terminal end **14** and its attachment end **16**. The strut **12** has strut sides **17** and **18** parallel to the slot **15**. As shown in the embodiment shown in FIG. 2, slot **15** extends entirely through the strut. As one example of such a slotted strut, for a stent thickness of 0.165mm (0.0065 inched) and a terminating strut measuring 0.305nm (0.012 inches) wide and approximately 1.27mm (0.05 inches) long, the slot 15 may be positioned toward the free end of the strut 12 and measure 0,152mm (0.006 inches) wide and 0.864nm (0.034 inches) long. As will be apparent to those of skill in the art, many other sizes and dimensions for a stent strut and/or a strut slot will also be appropriate. Instead of a slot, the strut can alternately have one or more indentations along the side surfaces of the strut which do not extend entirely through the strut.

FIG. 3 shows a preferred embodiment of a sleeve marker 20 prior to attachment to a stent strut. The marker **20** is formed in the shape of a hollow tube or sleeve shown, which is open at both ends as shown. The marker is formed from a radiopaque metal such as platinum, gold, tantalum, tungsten, iridium, etc., which is more radiopaque than that of the material from which the stent is formed. In a preferred embodiment, the material from which the marker is made is tantalum. The sleeve marker **20** is of a size and formed into a shape corresponding generally to that of the stent strut, and which can be slipped over and around the terminal end **14** of a strut **12** of the stent. For a strut having a generally rectangular cross-section such as the example described above, the sleeve marker will preferably also have a generally rectangular cross-section to match that of the strut. If the metal from which the sleeve marker **20** is to be formed is provided in round tube form, the marker tube material can be shaped to more closely fit the generally rectangular cross-section of the strut by, for example, choosing a mandrel of appropriate size and shape and forcing the marker tubing over the mandrel to form it into the desired rectangular shape.

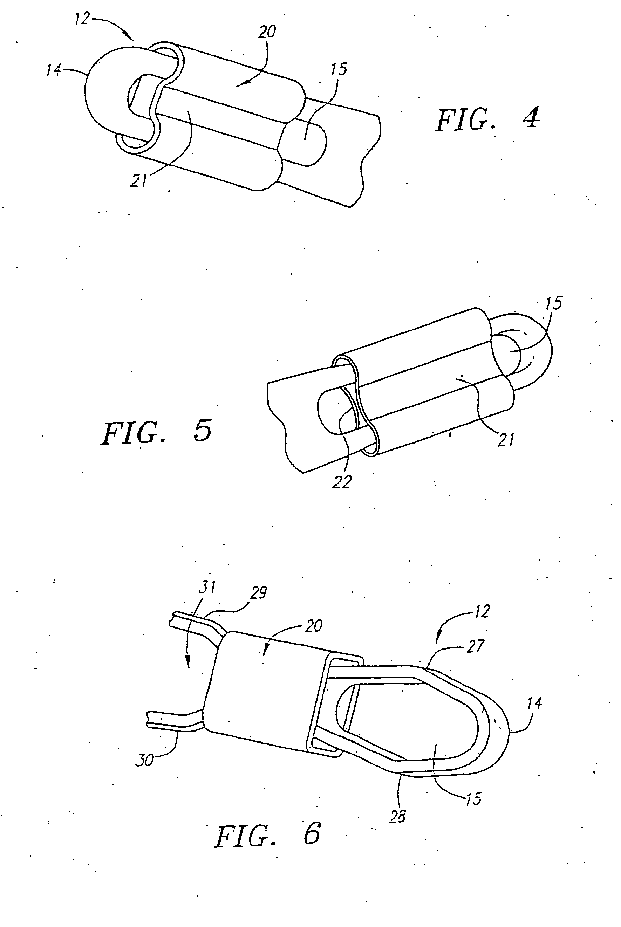

A marker **20** of appropriate size and shape relative to the stent strut 12 is provided and slid over and around a terminating stent strut **12**, and is centered over the slot **15**. The marker **20** is then permanently attached to the strut **12** by crimping, and specifically by using force to permanently deform the marker **20** so that part of the marker is pushed into the slot **15**. For example, the stent strut **12** may be placed in a holder with a forming die sized and shaped to firmly hold the strut. An aluminum dowel with a flat tip may be used to push the sides of the marker **20** into the fixture, deforming the marker and pushing part of it into the slot **15** on the strut **12**. As shown in FIG. 4, the resulting "crimped" side portion **21** of the marker extends below the surface level of the strut **12** into the slot 15, thereby providing positive attachment of the marker **20** to the strut by physically preventing the marker from sliding off the terminal end **14** of the strut due to its crimped shape. The marker can also be crimped on both sides, as shown in FIG. 5, by compressing and permanently deforming both of opposite sides **21** and **22** of the sleeve marker **20** down into the slot **15** and below the surface of the strut **12**. This results in a tighter fit of the sleeve marker **20** to the strut **12,** and provides a further means of preventing the marker from dislodging off the end of the strut. For a stent strut having one or more indentations, rather than a slot through the strut, the marker is similarly crimped into the indention(s) and is thereby permanently deformed to achieve positive attachment of the sleeve marker to the stent strut.

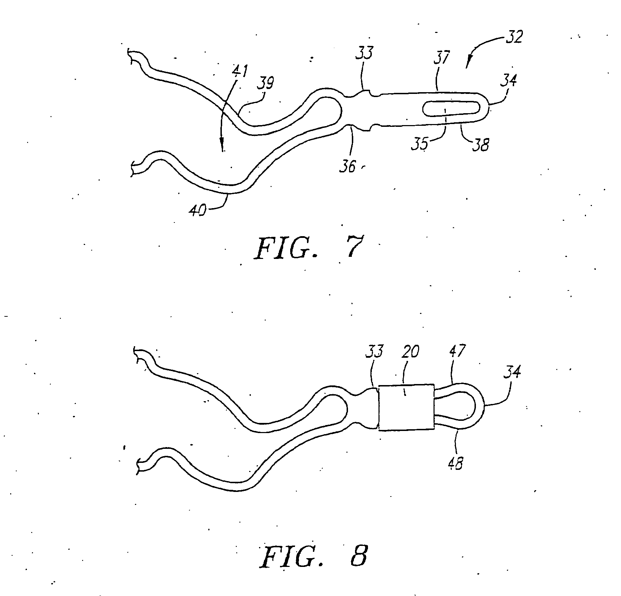

Alternately, a sleeve marker may be attached to a slotted stent strut formed of a shape memory material such as Nitinol by taking advantage of those shape memory properties. As shown in FIG. 6, in this embodiment the marker **20** is placed over and around a slotted terminating stent strut **12**, and the marker is pushed onto the strut past most or all of the slot **15** in strut **12** until the marker contacts a wider area of the stent adjacent to or formed by struts **29** and **30** of the adjoining open cell **31** of the stent. An expansion mandrel (not shown) is inserted into the slot **15** in the stent strut, resulting in outwardly deformed sides **27** and 28 of the strut. As an example, a 0.254mm (0.010 inch) mandrel may be used for the exemplary sleeve marker and slotted strut of the size described above. The stent slot is then heat-treated and the mandrel removed from the strut slot, resulting in a permanent deformation and widening of the strut slot which prevents the marker **20** from being slipped off the terminating strut. For example, the minimum outside dimension of the outwardly deformed sides **27** and **28** of the strut is greater than the maximum inside dimension of the marker **20**. This method of attaching the sleeve marker **20** to the stent strut has the advantage that the marker material is subjected to a reduced amount of forming during the process, since only the initial forming of the sleeve marker into the corresponding shape of the strut is required.

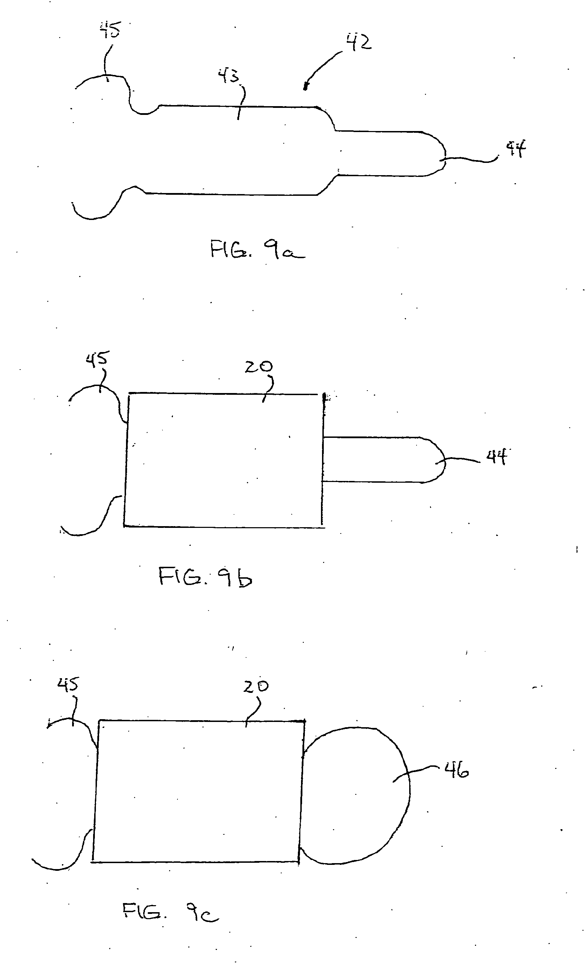

FIG. 7 shows a preferred embodiment of a slotted terminating stent strut **32** which has a free terminal end **34**, and stop member **33** at the opposite end **36** by which the strut is attached to the rest of the stent by struts **39** and **40** of the first open cell **41**. Sleeve marker **20** is pushed onto the stent strut **32** until the marker contacts stop member **33**, and then an expansion mandrel is forced into the slot **35** of strut **32** distal to marker **20**, resulting in outwardly deformed sides **47** and **48** of the strut and thereby widening of the strut slot distal to the marker, as shown in FIG. 8. The stent strut is then heat-treated, as discussed above, resulting in permanent deformation and widening of the strut slot and preventing removal of marker **20.**

The sleeve markers of this invention are preferably attached to struts at the ends of a stent, but could also be attached to any position along the stent having a terminating strut. Sleeve markers may be attached to multiple terminating struts of a stent. As an example, multiple sleeve markers may be attached to terminating struts evenly spaced around the entire periphery at each end of a stent, and in a preferred embodiment the sleeve markers are attached to alternating terminating struts. In a particularly preferred embodiment, five sleeve markers are attached to alternating terminating struts at each end of a mesh coil stent formed from the stent material shown in FIG 1. The marker material is more radiopaque than that of the stent. Therefore, after the stent with attached sleeve markers is loaded into a catheter and placed within a lumen in the body, the exact location of the stent, indicated by the attached markers, can be shown by fluoroscopy or other such techniques.

In an alternative embodiment, the sleeve marker is held in place on a terminating strut of a stent by other means of increasing the size of the terminal end of the strut distal to the sleeve marker after the marker has been slipped onto the strut. The terminating stent struts need not be slotted in this embodiment. For example, as shown in FIGS. 9a-9c, a weld ball may be formed on the end of the strut, the weld ball having a sufficient diameter to prevent removal of the sleeve marker from the stent strut. FIG. 9a shows a terminating strut **42** having a support portion **43,** an end **44**, and a stop member **45**. The support portion **43** has a width adapted to retain a sleeve marker **20**, and the stop member **45** has a size sufficient to prevent axial movement of the sleeve marker beyond the point of the stop member. Turning to FIG. 9b, a sleeve marker **20** is placed over the support portion to be held on the terminating strut. Once it is located, the end **44** of the terminating strut is melted to form a ball **46** having an outside diameter larger than the internal diameter of the sleeve marker, to thereby hold the sleeve marker in place, as shown in FIG. 9c. Melting of the end **44** of the terminating strut is preferably achieved by exposing the end material to a laser, although other methods are known to those of skill in the art. Further, although a ball-shape is preferred, the strut end **44** may be melted to form any shape capable of retaining the sleeve marker on the terminating strut.

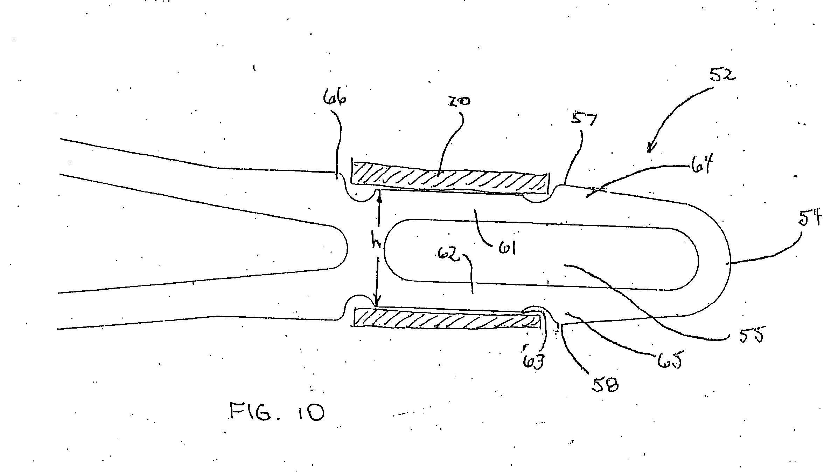

In the preferred embodiment shown in FIG. 10, a sleeve marker **20** (shown in cross-section) is held in place on a terminating stent strut **52** by forming the terminating strut having a width that cooperates to lock the sleeve marker **20** in a pocket **63** once it is placed over the terminating strut **52**. As shown in FIG. 10, the strut sides **57** and **58** are provided with narrowed sections **61** and **62,** the first narrowed section **61** being formed on the upper side of the upper strut side **57**, and the second narrowed section **62** being formed on the lower side of the lower strut side **58**. The narrowed sections **61** and **62** are formed by slightly thinning the exterior portion of the strut sides **57** and **58** at the same longitudinal location near the end **54** of the terminating strut to create a pocket **63** within which the sleeve marker **20** is located. The distance **h** between the upper edge of the first narrowed section **61** and the lower edge of the second narrowed section **62** generally corresponds to the inner dimension of the sleeve marker so that the sleeve marker fits snugly over the narrowed sections when the sleeve marker is placed on the terminating strut. The distal sections **64** and **65** of the terminating strut are not thinned, with the result that the width of the terminating strut **52** is slightly larger at the location of the distal sections **64** and **65** as compared to the width of the terminating strut at the location of the narrowed sections **61** and **62**, thereby creating the pocket **63** within which the sleeve marker is located. The sleeve marker **20** is placed on the terminating stent strut **52** by sliding it over the end **54** until the sleeve marker engages the narrowed sections **61** and **62** and is located in the pocket **63**. As the sleeve marker passes over the distal sections **64** and **65**, which have a slightly larger width than the internal dimension of the sleeve marker, the sleeve marker causes the distal sections **64** and **65** to compress slightly into the space formed by the slot **55**, thereby allowing the sleeve marker to pass over the distal sections **64** and **65** until coming to rest in the pocket **63**. After the sleeve marker passes over the distal sections **64** and **65,** the distal sections snap back into place, locking the sleeve marker in place. A ledge **66** is formed on the stent where the terminating strut **52** connects to the stent body. The ledge **66** acts as a stop to prevent movement of the sleeve marker past the location of the ledge **66**.

A variety of other methods can also be used to increase one or more of the dimensions of the terminal end of the strut distal to the sleeve marker by an amount sufficient to prevent removal of a marker placed onto the strut, or to provide a snap-fit mechanism on the distal end of the terminating strut, as will be apparent to those of skill in the art. For example, compression forces can be applied to the terminal end of the strut distal to the sleeve marker to permanently deform the strut's terminal end such that its width is increased beyond the width of the marker.

While preferred illustrative embodiments of the invention are described above, it will be apparent to one skilled in the art that various changes and modifications may be made thereto without departing from the invention, and which are accordingly within the scope of the invention taught herein.

## Claims

1. An endovascular stent (10) having a radiopaque marker (20) comprising:
a stent (10) of biocompatible material which is formed into struts surrounding open areas, said stent (10) having one or more elongated terminating struts (12) connected to the rest of the stent (10) at only one end (16) of said terminating strut (12); and
a marker (20) in the shape of a sleeve and formed of material which is more radiopaque than the material forming the stent (10), said marker (20) being placed on and surrounding a terminating strut (12) of the stent (10), and wherein said marker (20) is attached to the stent (10);
**characterised in that** the terminating strut (12) has a slot (15) through a portion of the strut (12) extending longitudinally along the length of the strut (12) but not extending to the terminal end (14) of the strut (12).

2. The stent of claim 1, wherein sides (17,18) of the strut (12) surrounding the slot (15) have been permanently deformed (27,28) to extend outward distal to the marker (20) to an extent sufficient to thereby prevent removal of the marker (20) from the strut (12).

3. The stent of claim 1, wherein the terminal end (14) of the strut (12) distal to the marker (20) has a cross-sectional dimension that is larger than a cross-sectional dimension of the marker (20) by an amount sufficient to inhibit removal of the marker (20) from the strut (12).

4. The stent of claim 1, wherein the marker (20) is attached to the stent (10) by a snap-fit mechanism.

5. The stent of claim 4, wherein said snap-fit mechanism includes a pocket (63) located on a portion of said terminating strut (52), said pocket (63) adapted to retain the marker (20) thereon.

6. The stent of claim 5, wherein said pocket (63) is defined by narrowed sections (61,62) of at least two portions (57,58) of the terminating strut (52).

7. The stent of claim 6, wherein said terminating strut (52) has a distal section (64,65) having a cross-sectional dimension that is larger than said narrowed section (61,62).

8. The stent of claim 7, wherein said marker (20) has an internal cross-sectional dimension (h) that is smaller than the cross-sectional dimension of the distal section (64,65) of said terminating strut (52).

9. The stent of claim 1, wherein the marker (20) has been permanently deformed so that part (21,22) of the marker (20) is pushed into the slot (15).

10. A method of attaching a radiopaque marker (20) to a stent (10), comprising:
providing a stent (10) formed of struts surrounding open areas, said stent having one or more elongated terminating struts (12), the terminating strut (12) having a slot (15) through a portion of the strut (12) extending longitudinally along the length of the strut (12) but not extending to the terminal end (14) of the strut (12);
providing a marker (20) in the shape of a sleeve, said marker (20) being made of a material that is more radiopaque than the material forming the stent (10); and
sliding the marker (20) over the terminal end (14) of the terminating strut (12) and advancing the marker (20) to a position surrounding the terminating strut (12).

11. The method of claim 10, further comprising attaching the marker (20) to the terminating strut (12) to thereby inhibit the marker (20) from sliding off the terminal end (14) of the strut (12).

12. The method of claim 11, wherein the terminating strut (52) has a snap-fit mechanism for attaching the marker (20) to the terminating strut (52) to thereby inhibit the marker (20) from sliding off the terminal end (54) of the strut (52).

13. The method of claim 12, wherein said snap-fit mechanism includes a pocket (63) located on a portion of said terminating strut (52), said pocket (63) adapted to retain the marker (20) thereon, said pocket (63) being defined by narrowed sections (61,62) of at least two portions (57,58) of the terminating strut (52), and wherein said terminating strut (52) has a distal section (64,65) having a cross-sectional dimension that is larger than said narrowed sections (61,62), and wherein said marker (20) has an internal cross-sectional dimension (h) that is smaller than the cross-sectional dimension of the distal section (64,65) of said terminating strut (52).

14. The method of claim 11, wherein the step of attaching the marker (20) to the terminating strut (12) comprises using force to permanently deform the marker (20) so that part (21,22) of the marker (20) is pushed into the slot (15).

15. The method of claim 11, wherein the step of attaching the marker (20) to the terminating strut (12) comprises permanently deforming (27,28) sides (17,18) of the strut (12) to extend outward distal to the marker (20) to an extent sufficient to thereby prevent removal of the marker (20) from the strut (12).

## Patentansprüche

1. Endovaskulärer Stent (10), der einen strahlenundurchlässigen Marker (20) aufweist, umfassend:
einen Stent (10) aus biokompatiblem Material, der von offene Flächen umgebenden Streben ausgebildet ist, wobei der Stent (10) eine oder mehrere längliche Endstreben (12) aufweist, die lediglich an einem Ende (16) der Endstrebe (12) mit dem Rest des Stents (10) verbunden sind; und
einen Marker (20), der die Gestalt einer Hülse aufweist und aus einem Material gebildet ist, welches strahlenundurchlässiger ist als das Material, aus welchem der Stent (10) gebildet ist, wobei der Marker (20) auf einer Endstrebe (12) des Stents (10) angeordnet ist und diese umgibt, und wobei der Marker (20) am Stent (10) befestigt ist;
**dadurch gekennzeichnet, dass** die Endstrebe (12) durch einen Abschnitt der Strebe (12) hindurch einen Schlitz (15) aufweist, der sich in Längsrichtung entlang der Länge der Strebe (12) erstreckt, sich jedoch nicht bis zum abschließenden Ende (14) der Strebe (12) erstreckt.

2. Stent gemäß Anspruch 1, bei welchem Seiten (17, 18) der Strebe (12), die den Schlitz (15) umgeben, dauerhaft verformt (27, 28) worden sind, um sich distal zum Marker (20) um ein Maß nach außen zu erstrecken, welches ausreicht, um **dadurch** das Entfernen des Markers (20) von der Strebe (12) zu verhindern.

3. Stent gemäß Anspruch 1, bei welchem das abschließende Ende (14) der Strebe (12) distal zum Marker (20) eine Querschnittsbemessung aufweist, die größer ist als eine Querschnittsbemessung des Markers (20), und zwar um einen Betrag, der ausreicht, um das Entfernen des Markers (20) von der Strebe (12) zu verhindern.

4. Stent gemäß Anspruch 1, bei welchem der Marker (20) mittels eines Schnappbefestigungsmechanismus am Stent (10) befestigt ist.

5. Stent gemäß Anspruch 4, bei welchem der Schnappbefestigungsmechanismus eine Tasche (63) einschließt, die auf einem Abschnitt der Endstrebe (52) angeordnet ist, wobei die Tasche (63) dazu angepasst ist, den Marker (20) auf dieser zurückzuhalten.

6. Stent gemäß Anspruch 5, bei welchem die Tasche (63) durch verengte Abschnitte (61, 62) von wenigstens zwei Abschnitten (57, 58) der Endstrebe (52) festgelegt ist.

7. Stent gemäß Anspruch 6, bei welchem die Endstrebe (52) einen distalen Abschnitt (64, 65) aufweist, der eine größere Querschnittsbemessung aufweist als der verengte Abschnitt (61, 62).

8. Stent gemäß Anspruch 7, bei welchem der Marker (20) eine innere Querschnittsbemessung (h) aufweist, die kleiner ist als die Querschnittsbemessung des distalen Abschnitts (64, 65) der Endstrebe (52).

9. Stent gemäß Anspruch 1, bei welchem der Marker (20) derart dauerhaft verformt worden ist, dass ein Teil (21, 22) des Markers (20) in den Schlitz (15) geschoben ist.

10. Verfahren zum Befestigen eines strahlenundurchlässigen Markers (20) an einem Stent (10), umfassend:
Vorsehen eines Stents (10), der von offene Flächen umgebenden Streben gebildet ist, wobei der Stent (10) eine oder mehrere längliche Endstreben (12) aufweist, wobei die Endstrebe (12) durch einen Abschnitt der Strebe (12) hindurch einen Schlitz (15) aufweist, der sich in Längsrichtung entlang der Länge der Strebe (12) erstreckt, sich jedoch nicht bis zum abschließenden Ende (14) der Strebe (12) erstreckt;
Vorsehen eines Markers (20) in der Gestalt einer Hülse, wobei der Marker (20) aus einem Material gebildet ist, welches strahlenundurchlässiger ist als das Material, welches des Stent (10) bildet; und
Schieben des Markers (20) über das abschließende Ende (14) der Endstrebe (12) und Vorrücken des Markers (20) an eine die Endstrebe (12) umgebende Position.

11. Verfahren gemäß Anspruch 10, welches ferner das Befestigen des Markers (20) an der Endstrebe (12) umfasst, um **dadurch** zu verhindern, dass der Marker (20) vom abschließenden Ende (14) der Strebe (12) wegrutscht.

12. Verfahren gemäß Anspruch 11, bei welchem die Endstrebe (52) einen Schnappbefestigungsmechanismus aufweist, um den Marker (20) an der Endstrebe (52) zu befestigen, um **dadurch** zu verhindern, dass der Marker (20) vom abschließenden Ende (54) der Strebe (52) wegrutscht.

13. Verfahren gemäß Anspruch 12, bei welchem der Schnappbefestigungsmechanismus eine Tasche (63) einschließt, die auf einem Abschnitt der Endstrebe (52) angeordnet ist, wobei die Tasche (63) dazu angepasst ist, den Marker (20) auf dieser zurückzuhalten, wobei die Tasche (63) durch verengte Abschnitte (61, 62) von wenigstens zwei Abschnitten (57, 58) der Endstrebe (52) festgelegt wird und wobei die Endstrebe (52) einen distalen Abschnitt (64, 65) aufweist, der eine größere Querschnittsbemessung aufweist als die verengten Abschnitte (61, 62), und wobei der Marker (20) eine innere Querschnittsbemessung (h) aufweist, die kleiner ist als die Querschnittsbemessung des distalen Abschnitts (64, 65) der Endstrebe (52).

14. Verfahren gemäß Anspruch 11, bei welchem der Schritt des Befestigens des Markers (20) an der Endstrebe (12) das Verwenden einer Kraft umfasst, um den Marker (20) derart dauerhaft zu verformen, dass der Teil (21, 22) des Markers (20) in den Schlitz (15) geschoben wird.

15. Verfahren gemäß Anspruch 11, bei welchem Schritt des Befestigens des Markers (20) an der Endstrebe (12) das dauerhafte Verformen (27, 28) von Seiten (17, 18) der Strebe (12) umfasst, so dass sie sich distal zum Marker (20) in einem Maß nach außen erstrecken, welches ausreicht, um **dadurch** das Entfernen des Markers (20) von der Strebe (12) zu verhindern.

## Revendications

1. Extenseur endovasculaire (10) comportant un marqueur radio-opaque (20) comprenant :
un extenseur (10) constitué d'un matériau biocompatible qui est formé en entretoises entourant des zones ouvertes, ledit extenseur (10) comportant une ou plusieurs entretoises de terminaison allongées (12) reliées au reste de l'extenseur (10) au niveau d'une seule extrémité (16) de ladite entretoise de terminaison (12) ; et
un marqueur (20) sous la forme d'un manchon et formé d'un matériau qui est plus radio-opaque que le matériau formant l'extenseur (10), ledit marqueur (20) étant placé sur et autour d'une entretoise de terminaison (12) de l'extenseur (10), et où ledit marqueur (20) est fixé à l'extenseur (10) ;
**caractérisé en ce que** l'entretoise de terminaison (12) présente une fente (15) au travers d'une partie de l'entretoise (12) s'étendant longitudinalement sur la longueur de l'entretoise (12) mais ne s'étendant pas jusqu'à l'extrémité terminale (14) de l'entretoise (12).

2. Extenseur selon la revendication 1, dans lequel les côtés (17, 18) de l'entretoise (12) entourant la fente (15) ont été déformés de façon permanente (27, 28) pour s'étendre vers l'extérieur de façon distale par rapport au marqueur (20) dans une mesure suffisante pour empêcher ainsi l'enlèvement du marqueur (20) depuis l'entretoise (12).

3. Extenseur selon la revendication 1, dans lequel l'extrémité terminale (14) de l'entretoise (12) distale par rapport au marqueur (20) présente une dimension en section transversale qui est supérieure à une dimension en section transversale du marqueur (20) d'une valeur suffisante pour empêcher l'enlèvement du marqueur (20) de l'entretoise (12).

4. Extenseur selon la revendication 1, dans lequel le marqueur (20) est fixé à l'extenseur (10) par un mécanisme de montage par encliquetage.

5. Extenseur selon la revendication 4, dans lequel ledit mécanisme de montage par encliquetage comprend une poche (63) située sur une partie de ladite entretoise de terminaison (52), ladite poche (63) étant conçue pour retenir le marqueur (20) sur celle-ci.

6. Extenseur selon la revendication 5, dans lequel ladite poche (63) est définie par des sections rétrécies (61, 62) d'au moins deux parties (57, 58) de l'entretoise de terminaison (52).

7. Extenseur selon la revendication 6, dans lequel ladite entretoise de terminaison (52) présente une section distale (64, 65) ayant une dimension en section transversale qui est supérieure à ladite section rétrécie (61, 62).

8. Extenseur selon la revendication 7, dans lequel ledit marqueur (20) présente une dimension en section transversale interne (h) qui est inférieure à la dimension en section transversale de la section distale (64, 65) de ladite entretoise de terminaison (52).

9. Extenseur selon la revendication 1, dans lequel le marqueur (20) a été déformé de façon permanente de telle sorte qu'une partie (21, 22) du marqueur (20) est poussée dans la fente (15).

10. Procédé de fixation d'un marqueur radio-opaque (20) sur un extenseur (10), comprenant :
la fourniture d'un extenseur (10) formé d'entretoises entourant des zones ouvertes, ledit extenseur ayant une ou plusieurs entretoises de terminaison allongées (12), l'entretoise de terminaison (12) comportant une fente (15) au travers d'une partie de l'entretoise (12) s'étendant longitudinalement sur la longueur de l'entretoise (12) mais ne s'étendant pas jusqu'à l'extrémité terminale (14) de l'entretoise (12),
la fourniture d'un marqueur (20) sous la forme d'un manchon, ledit marqueur (20) étant constitué d'un matériau qui est plus radio-opaque que le matériau formant l'extenseur (10), et
le fait de faire coulisser le marqueur (20) sur l'extrémité terminale (14) de l'entretoise de terminaison (12) et de faire avancer le marqueur (20) jusqu'à une position entourant l'entretoise de terminaison (12).

11. Procédé selon la revendication 10, comprenant en outre la fixation du marqueur (20) à l'entretoise de terminaison (12) pour empêcher ainsi le marqueur (20) de glisser en dehors de l'extrémité terminale (14) de l'entretoise (12).

12. Procédé selon la revendication 11, dans lequel l'entretoise de terminaison (52) comporte un mécanisme de montage par encliquetage destiné à fixer le marqueur (20) à l'entretoise de terminaison (52) pour empêcher ainsi le marqueur (20) de glisser en dehors de l'extrémité terminale (54) de l'entretoise (52).

13. Procédé selon la revendication 12, dans lequel ledit mécanisme de montage par encliquetage comprend une poche (63) située sur une partie de ladite entretoise de terminaison (52), ladite poche (63) étant conçue pour retenir le marqueur (20) sur celle-ci, ladite poche (63) étant définie par des sections rétrécies (61, 62) d'au moins deux parties (57, 58) de l'entretoise de terminaison (52), et dans lequel ladite entretoise de terminaison (52) comporte une section distale (64, 65) ayant une dimension en section transversale qui est supérieure à celle desdites sections rétrécies (61, 62), et dans lequel ledit marqueur (20) présente une dimension en section transversale interne (h) qui est inférieure à la dimension en section transversale de la section distale (64, 65) de ladite entretoise de terminaison (52).

14. Procédé selon la revendication 11, dans lequel l'étape de fixation du marqueur (20) à l'entretoise de terminaison (12) comprend l'utilisation d'une force pour déformer de façon permanente le marqueur (20) de telle sorte qu'une partie (21, 22) du marqueur (20) soit poussée dans la fente (15).

15. Procédé selon la revendication 11, dans lequel l'étape de fixation du marqueur (20) à l'entretoise de terminaison (12) comprend une déformation permanente (27, 28) des côtés (17, 18) de l'entretoise (12) pour s'étendre vers l'extérieur de façon distale par rapport au marqueur (20) dans une mesure suffisante pour empêcher l'enlèvement du marqueur (20) de l'entretoise (12).
